# EUROPEAN PATENT APPLICATION

(11) **EP 4 696 239 A1**
(43) Date of publication of application: **18.02.2026**
(21) Application number: 24194543.5
(22) Date of filing: 14.08.2024
(51) Int. Cl.: A61B 6/00, A61B 34/20

(54) **TRACKABLE MEDICAL IMAGING DEVICE**

(71) Applicant: Stryker European Operations Limited, Carrigtwohill, Co. Cork T45 HX08 (IE)
(72) Inventor: LACKERMEIER, Lea, 79106 Freiburg im Breisgau (DE); HORNECKER, Patrick, 79356 Eichstetten (DE)
(74) Representative: Röthinger, Rainer

(57) **Abstract**

A medical imaging device 100 is provided. The device 100 comprises a non-stationary housing 110, an optical tracker 120 configured to be detected by a camera system 200 and comprising a plurality of optically detectable markers 122 attached to the housing 110, at least one storage 124 configured to store calibration information relating to the optical tracker 120, and an optical communication interface 130 configured to output to the camera system 200 one or more optical communication signals indicative of the calibration information.

## Description

### Technical Field

The present disclosure generally relates to medical imaging. In particular, a medical imaging device with an optical tracker, a system comprising the medical imaging device, associated methods and a computer program product are presented.

### Background

Medical imaging devices capable of generating high-resolution patient images have a variety of applications in modern medicine. For example, the patient images can be used for pre- and intra-operatively analyzing a state of a patient, but also for surgical planning and assisting navigation during a surgical intervention. As is well known, navigated surgery based on patient images improves safety and efficiency in minimal invasive and other surgical procedures. For this reason, surgical navigation systems are becoming increasingly popular for all types of surgical procedures.

At some point, the patient images generated by a medical imaging device have to be communicated to the surgical navigation system. EP 2 838 433 B1 discloses an imaging system comprising a gantry connected to a navigation system using a wireless communications system for transmitting the patient images and computer models. The wireless communications system is configured to use an IEEE 802.11 a/b/g/n transfer protocol. The underlying communication connection can be configured as either an ad-hoc point-to-point network or using access points through a router.

Known optical navigation systems comprise a tracking camera that continuously tracks within a tracking coordinate system the positions of one or more optical trackers attached to a patient and to surgical instruments. It is also known to attach trackers to medical imaging devices having non-stationary housings so that their positions can be tracked as well.

Typical optical trackers comprises a plurality of optical markers, such as light emitting diodes, arranged in a predefined pattern. To properly identify each tracker in the image information generated by the tracking camera, the navigation system needs to know the predefined pattern in which the optical markers of a particular tracker is arranged. The pattern is stored by the navigation system in the form of calibration information. Since the marker pattern of an optical tracker is typically predefined at the manufacturing stage, the corresponding calibration information can be provided to the navigation system at the manufacturing stage as well.

### Summary

There is a need for a technique for efficiently providing calibration information relating to an optical tracker attached to a medical imaging device.

According to a first aspect, a medical imaging device is provided. The medical imaging device comprises a non-stationary housing, an optical tracker configured to be detected by a camera system and comprising a plurality of optically detectable markers attached to the housing, at least one storage configured to store calibration information relating to the optical tracker, and an optical communication interface configured to output to the camera system at least one first optical communication signal indicative of the calibration information.

The first communication signal may occur at any point in time of a possible communication sequence. In other words, it may occur before or after another ("second") communication signal.

The camera system may be comprised by a surgical navigation system. The surgical navigation system may be configured to detect the first and any other communication signal in image information generated by the camera system. In other variants, the camera system may comprise a dedicated receiver for the first and any further communication signal.

The medical imaging device may be a stationary or a mobile device. The medical imaging device may have a base. The non-stationary housing may be movable relative to the base. The base may be mobile (e.g., provided with wheels) or stationary. The housing of the medical image device may be, or belong to, a gantry of the medical imaging device. As one example, the Stryker ^{®} Airo^{®} TruCT^{®} imaging device comprises a base and a ring-shaped gantry carried by the base.

The optical tracker attached to the housing of the medical imaging device may comprise three, four, five, six or more optically detectable markers. The markers may be mechanically attached to the housing. Mechanically attaching the markers to the housing may prevent movement of the markers relative to the housing and relative to each other. The markers may be detectable in at least one of the visible and the infrared spectrum. The markers may be, or may comprise, one or more active markers (e.g., comprising light-emitting diodes, LEDs) and/or one or more passive markers (e.g., light-reflecting elements).

The optical communication interface may comprise at least one LED or other light source configured to output optical communication signals (e.g., in the visible or the infrared spectrum). In some implementations, the optical communication interface may further be configured to receive optical communication signals. In the latter case, the optical communication interface is configured as a bi-directional interface, but it can also be realized as a uni-directional interface only capable of outputting optical communication signals. The output and/or received optical communication signals may be further processed. The signal processing may comprise at least one of signal conversion, amplification and decoding. Multiple signal processing steps may be performed in sequence and in any suitable order. The optical communication interface and, optionally, the camera system or another communication counterpart of the optical communication interface may comprise electronic components configured for performing the signal processing, i.e., at least one of converting, amplifying and decoding the signal. The optical communication interface and, optionally, its counterpart may further comprise at least one electronic component configured for at least one of signal timing /signal synchronization, power supply and performing or initiating regular calibration cycles.

The optical communication interface may enable communication from the medical imaging device to the camera system and, optionally, in the opposite direction. The optical communication interface may be used in parallel to a wire-based and/or a radio interface for data transfer between the medical imaging device and the camera system (e.g., for redundancy purposes or for taking over at least some of data transfers). Alternatively, the optical communication interface may be utilized to completely replace any other data transfer interfaces between the medical imaging device and the camera system.

The calibration information may be indicative of at least one of relative positions between the optically detectable markers and locations at which the optically detectable markers are attached to the housing. The relative positions and locations may be determined during manufacturing, e.g., based on manufacturing data. The manufacturing data may comprise dimensions of the housing and the locations of the markers thereon. The relative positions and locations may be stored in the at least one storage already during manufacturing or at a later point in time (e.g., during an on-site calibration procedure). In some variants, the relative positions between the markers and/or their locations on the housing may be determined based on image data from the camera system, such as for verifying or updating previously acquired calibration information received via the first optical communication signal(e.g., in the form of regular quality checks on a yearly or half yearly basis, for example).

The medical imaging device may define an image volume. In such a case, the calibration information may be indicative of a positional relationship between the optical tracker attached to the housing and the image volume. The positional relationship between the optical tracker and the image volume may be initially determined during manufacturing of the medical imaging device. A calibration phantom device may be utilized for determining the positional relationship between the optical tracker and the image volume. The positional relationship between the optical tracker and the image volume may substantially remain fixed and may be verified via quality checks (e.g., on a regular basis, such as once per year).

Each of the optical tracker and the image volume may define a respective coordinate system. In some variants, the positional relationship between the optical tracker and the image volume can be defined based on a coordinate transformation between the coordinate system defined by the optical tracker and the coordinate system defined by the image volume. The respective coordinate system may be determined based on manufacturing data and/or image data acquired by the imaging device and the camera system.

The calibration information may additionally comprise one or more of a focal length of the medical imaging device, a static iso- or rotation-center of the medical imaging device, and a geometric relation of the coordinate system defined by the image volume to the iso- or rotation-center.

In some variants, the medical imaging device may define a coordinate system. In such variants, the coordinate transformation between the coordinate system defined by the optical tracker and the coordinate system defined by the image volume can be based on coordinate transformations between the coordinate systems defined by the medical imaging device and the optical tracker, and between the coordinate systems defined by the medical imaging device and the image volume. The coordinate system defined by the medical imaging device may be used as a global coordinate system, since the coordinate system is typically not subject to any change (i.e., fixed). The calibration information may indicate the locations of the markers in the coordinate system defined by the medical imaging device.

The transformation between the coordinate systems defined by the medical imaging device and the optical tracker may be determined based on image data generated with the medical imaging device and may be indicative of a calibration phantom device. In some instances, the transformation between the coordinate systems defined by the medical imaging device and the optical tracker may be determined based on manufacturing data, e.g., predefined positions of the markers of the tracker on the housing of the medical imaging device.

One or more of the coordinate transformations discussed herein may be stored as calibration information relating to the optical tracker in the at least one storage of the medical imaging device. The transformations may generally remain fixed (e.g., unless of a mechanical damage occurs). One or more of the transformations may be checked or verified (e.g., via regular quality checks). The calibration information may further comprise at least one of a calibration matrix indicative of a combination of coordinate transformations, a calibration date, a calibration direction, and a current state of active optical markers (e.g., on/off or operative/defect, etc.)

The medial imaging device may be configured to transit a workflow. For example, the medical imaging device may be configured as a scanning device capable of transiting from a scan readiness state to a scan finished state. In some variants, the at least one first optical communication signal may further be indicative of a workflow state of the medical imaging device. For example, an operation frequency of at least one LED as an exemplary element of the optical communication interface can be modulated to signal different workflow states of the medical imaging device, such as one of a scan readiness state and a scan finished state to the camera system (and, thus, the surgical navigation system comprising the camera system).

In some variants, the optical communication interface of the medical imaging device may be configured to receive at least one second optical communication signal, e.g., from a transmitter co-located with the camera system. The at least one second optical communication signal may be indicative of at least one of a visibility of at least one of the plurality of optically detectable markers, and an instruction to the medical imaging device. The instruction may, for example, trigger a workflow of the medical imaging device. The instruction may be based at least in part on the workflow state of the medical imaging device indicated in the at least one first optical communication signal.

In some variants, the medical imaging device may be configured to trigger a workflow in response to the at least one second optical communication signal. The triggered workflow may comprise at least one of a calibration or a re-calibration of the medical imaging device, and a generation of image data with the medical imaging device (e.g., performing one or more scans with the medical imaging device). For example, if the medical imaging device is in a scan readiness state and the at least one second optical communication signal comprises an instruction to trigger a scan, the medical imaging device may trigger the scan (as an exemplary workflow) upon processing of the at least one second optical communication signal. In another example, a re-calibration may be triggered, e.g., depending on the last calibration date.

In some variants, the at least one second optical communication signal may be indicative of a position of the optical tracker within a coordinate system defined by the camera system. In such variants, the workflow may comprise determining a coordinate transformation between the coordinate system defined by the image volume and the coordinate system defined by the camera system based at least on the at least one second optical communication signal and the positional relationship between the optical tracker and the image volume. Alternatively, a processor coupled to the camera system may determine the coordinate transformation between the coordinate system defined by the image volume and the coordinate system of the camera system, and the at least one second optical communication signal may be indicative thereof. In some implementations, the coordinate system of the camera system may be used as a global coordinate system, alternatively to the coordinate system defined by the medical imaging device.

In some variants, the medical imaging device may further comprise a first controller coupled to the storage and configured to control the optical communication interface. The medical imaging device may further comprise a second controller coupled to at least one of the storage and the first controller. The second controller may be configured to control imaging of the medical imaging device. The first controller and the storage may form a computation unit (e.g., a so-called "smart unit"). The second controller may be, or may form a part of, a core computation unit of the medical imaging device. In this case the "smart unit" may maintain a connection to the second controller to ensure data exchange checks (e.g., relating to a calibration date or checksums) when exchanging data between the first and the second controller. In other implementations, the storage may also form a part of the core computation unit of the medical imaging device. In this case, all functions of the smart unit described above may be an integral part of the core computation unit of the medical imaging device. The first controller may be configured to at least one of process the at least one second optical communication signal received via the optical communication interface and transmit the received or processed at least one second optical communication signal to the second controller.

The medical imaging device may further comprise a power source configured to power the medical imaging device and at least one of the optical tracker and the optical communication interface. The power source may comprise an energy transmitting element configured to inductively transmit power (e.g., in the form of one or more coils). In some implementations, at least one of the optical tracker and the optical communication interface may be powered via one or more additional power sources, or via battery.

The medical imaging device may be a C-arm or computer tomography, CT, scanner. The housing of the medical imaging device may be, or comprise, a gantry in form of a C-arm or an O-arm. The housing may be movable in multiple directions, thus enabling multiple scanning directions for the medical imaging device. In some variants, the storage may comprise calibration information for each of the scanning directions.

The housing may comprise a plurality of surfaces. At least two of the surfaces may comprise at least one of the plurality of the optically detectable markers. For example, at least two of a front surface, a back surface and a top surface of a gantry may each comprise one or more markers. Consequently, visibility of at least one marker from every viewing direction is ensured. In this way, calibration and navigation for every scanning direction is enabled.

According to a second aspect, a system is provided. The system comprises the medical imaging device as explained herein and a camera system. The camera system is configured to detect the optical tracker and to communicate with the optical communication interface. The system further comprises a processor coupled to the camera system and configured for processing the at least one first optical communication signal indicative of the calibration information, wherein processing of the at least one first optical communication signal comprises determining at least one of a calibration state of the medical imaging device, a coordinate transformation between the coordinate system defined by the camera system and a coordinate system defined by an image volume, and at least one second optical communication signal indicative of an instruction to the medical imaging device.

The camera system may comprise at least one camera or multiple cameras (e.g., in the form of a stereo camera). The camera system may have a sensitivity in the visible or the infrared spectrum. A transmitter for the at least one second optical communication signal may be co-located with (e.g., integrated into a housing of) the camera system.

According to a third aspect, a method for operating a medical imaging device is provided. The medical imaging device comprises a non-stationary housing; an optical tracker configured to be detected by a camera system and comprising a plurality of optically detectable markers attached to the housing; at least one storage configured to store calibration information relating to the optical tracker; and an optical communication interface configured to output to the camera system at least one first optical communication signal indicative of the calibration information. The method comprises outputting, via the optical communication interface, the at least one first optical communication signal to the camera system.

The method according to the third aspect may further comprise receiving, via the optical communication interface, at least one second optical communication signal. The method may further comprise triggering a workflow based at least on the at least one second optical communication signal. The workflow may comprise at least one of a calibration, a re-calibration and a scan for generating a patient image as described with reference to the medical imaging device above.

According to a fourth aspect, a method for operating a camera system is provided. The camera system comprises at least one camera configured to detect an optical tracker of a medical imaging device and an optical communication interface configured to receive, from the medical imaging device, at least one first optical communication signal indicative of calibration information relating to an optical tracker attached to a non-stationary housing of the medical imaging device. The method comprises receiving, via the optical communication interface, the at least one first optical communication signal from the medical imaging device, and processing the at least one first optical communication signal.

Processing the at least one first optical communication signal may comprise determining at least one of a calibration state of the medical imaging device, a coordinate transformation between a coordinate system defined by the camera system and a coordinate system defined by an image volume of the medical imaging device, and at least one second optical communication signal indicative of an instruction to the medical imaging device.

According to a fifth aspect, a computer program product is provided. The computer program product comprises instructions that, when executed on at least one processor, cause the at least one processor to carry out any of the methods described herein.

### Brief Description of the Drawings

Further details, advantages and aspects of the present disclosure will become apparent from the following embodiments taken in conjunction with the drawings, wherein:
- Fig. 1: schematically shows a first medical imaging/tracking system comprising a first variant of a medical imaging device and a camera system;
- Fig. 2: is a flow diagram schematically illustrating a method for operating a medical imaging device;
- Fig. 3: is a flow diagram schematically illustrating a method for operating a camera system;
- Fig. 4: schematically shows the system of Fig. 1, wherein the medical imaging device is rotated compared to Fig. 1; and
- Fig. 5: schematically shows a second medical imaging/tracking system comprising the camera system of Fig. 1 and a second variant of a medical imaging device.

### Detailed Description

In the following description of exemplary embodiments, the same reference numerals are used to denote the same or similar components.

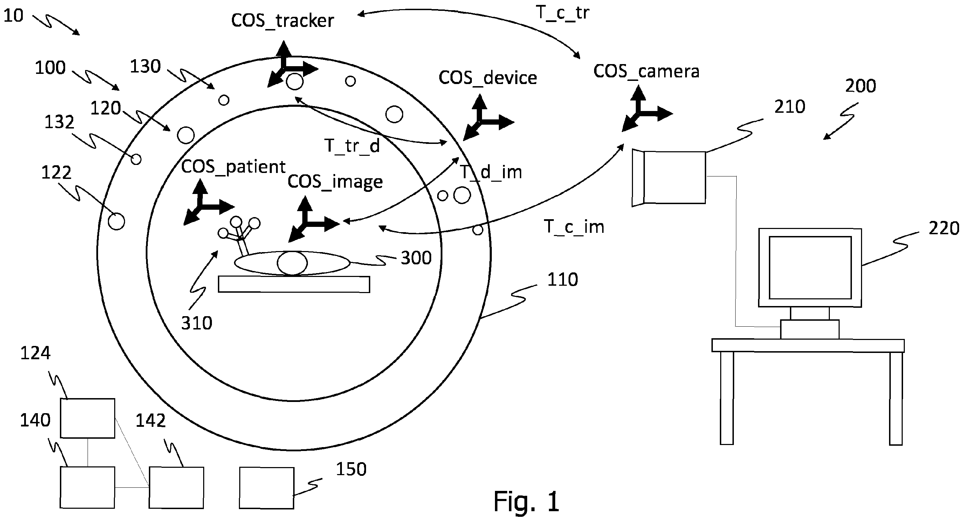

Fig. 1 schematically illustrates a medical imaging/tracking system 10 that comprises a first variant of a medical imaging device 100 as well as a camera system 200. The medical imaging device 100 is configured to intra-operatively generate images of a patient 300. As illustrated in Fig. 1, the patient 300 is provided with a patient tracker 310 that is detectable by the camera system 200. In some variants, the imaging device 100 may be based on the commercially available Stryker^{®} Airo^{®} TruCT^{®} imaging device. The camera system 200 may belong to, or may be in communication with, a surgical navigation system.

The medical imaging device 100 illustrated in Fig. 1 is realized as a CT scanner. It includes a gantry configured as an O-arm with a rigid, ring-shaped housing 110. The housing 110 is non-stationary with respect to a room in which the medical imaging device 100 is arranged. As an example, housing 110 may be moveable relative to or together with a base (not shown) of the medical imaging device 100. An imaging unit with an X-ray source and an X-ray detector (not shown) is arranged to spin freely within the housing 110. The imaging device 100 defines a coordinate system COS_device that may be anchored relative to (e.g., at) the rigid housing 110.

The imaging device 100 further comprises a tracker 120 detectable by the camera system 200. The tracker 120 is formed by a plurality of optically detectable markers 122 attached at least to a front side of the housing 110. One can say that the markers 122 form a rigid body that constitutes the tracker 120.

Each marker 122 of the plurality of optically detectable markers 122 has a fixed, predefined location on the housing 110. In some variants, the markers 122 are active light-emitting elements such as LEDs (e.g., infrared LEDs). Compared to passive markers, the active markers 122 provide the advantage that they can still be tracked by the camera system 200 when the housing 110 of the imaging device 100 (and, thus, the markers 122) are draped. Therefore, the need to drape the patient 300 is omitted and as a result, stress on the patient 300 is reduced. Further, the duration the patient 300 needs to be located in the medical imaging device 100 can be reduced, since the draping of the housing 110 can be prepared in advance.

In the variant shown in Fig. 1, the optically detectable markers 122 are distributed in a non-regular pattern about the upper half of the ring-shaped front side of the housing 110. As illustrated in Fig. 1, the tracker 120 defines a tracker coordinate system COS_tracker. The positions of the markers 122 may be expressed in this tracker coordinate system COS_tracker and constitute calibration information. This calibration information is stored (e.g., at the manufacturing stage or at a later re-calibration stage) in a local storage 124 of the imaging device 100.

Since the locations of the markers 122 on the housing 110 are fixed and the housing 110 is rigid, a coordinate transformation T_tr_d between the coordinate system defined by the tracker 120, i.e., COS_tracker, and the coordinate system defined by the medical imaging device 100, i.e., COS_device, can be defined. As an example, this coordinate transformation can be determined based on manufacturing data, i.e., based on the predefined locations of the markers 122 and the dimensions of the medical imaging device 100, in particular of the housing 110.

The medical imaging device 100 defines an image volume. The image volume has its own coordinate system COS_image in which the image information is expressed (e.g., in a DICOM format). Generally, the image volume has a fixed relation to an iso- and rotation-center of the medical imaging device 100 that can be defined, for example, by a radiation source and a radiation detector configured to rotate within the housing 110 and around the iso- and rotation-center. The fixed relation between the image volume to the iso- and rotation-center of the medical imaging device 100 may be determined based at least on one of manufacturing data of the medical imaging device 100 and image data generated with the medical imaging device 100 and indicative of a calibration phantom device (not shown). Based on the fixed relation between the image volume and the iso- and rotation-center, a coordinate transformation T_d_im between the coordinate system defined by the imaging device 100 (i.e., COS_device) and the coordinate system defined by the image volume (i.e., COS_image) may be determined.

Based on the coordinate transformations T_tr_d and T_d_im, a spatial relation between the tracker 120 and the image volume can be determined. In other words, the medical imaging device 100 is calibrated based thereon. The coordinate transformations T_tr_d and T_d_im are generally fixed, with the exception of material wear or shocks on the imaging device 100.

The coordinate transformations T_tr_d and T_d_im are stored as exemplary further calibration information (e.g., at the manufacturing stage or at a later re-calibration stage) in the storage 124. Since the calibration information is saved in the storage 124 and generally fixed, the coordinate transformations T_tr_d and T_d_im have only to be verified in predefined maintenance intervals, e.g., once or twice a year, after an initial determination during manufacturing. Alternatively, the transformations T_tr_d and T_d_im may be verified when a shock on the medical imaging device 100, in particular the housing 110 has occurred.

Compared to calibration techniques in which an optical tracker (not shown) is used that has no predefined, fixed relation to the housing 110 of the medical imaging device 100 (e.g., that is newly attached before every surgical procedure), the complexity and the time needed for calibrating the medical imaging device 100 is reduced when using the tracker 120 with markers 122 having fixed, predefined locations on the housing 110 of the medical imaging device 100 since the transformations T_tr_d and T_d_im are calibrated in advance and can be retrieved as calibration information from the storage 124 of the medical imaging device 100.

The medical imaging device 100 shown in Fig. 1 further comprises an optical communication interface 130 comprising multiple LEDs 132 located on the front side of the housing 110 of the medical imaging device 100. The LEDs 132 of the interface 130 are distributed about the upper half of the front side of the housing 110 in a non-regular pattern. Alternatively, the LEDs 132 may be distributed with a regular pattern, e.g., with constant distances from each other. The LEDs 132 may be configured to emit infrared light for outputting optical communication signals, i.e., for optical data transfer, indicative of the one more items of calibration information stored in the local storage 124. The optical communication interface 130 can further be configured to receive optical communication signals. To this end, the interface 130 may comprise one or more light receivers (e.g., sensitive in the infrared spectrum). The provision of the optical communication interface 130 enables a communication with the camera system 200 that is independent of a workflow state of the imaging device 100 and conventional communication protocols such as Ethernet or any other IEEE-defined communication protocols.

The signal emitted by the communication interface 130 may comprise or may be a pre-signal in form of a start signal or a handshake signal. A receiver of this signal (e.g., the camera system 200 or a dedicated additional receiver) and the optical communication interface 130 of the medical imaging device 100 may use the same communication protocol. The camera system 200 and the optical communication interface 130 of the medical imaging device 100 may communicate via signals known to each other and, optionally, to a surgical navigation system. In some variants, the camera system 200 may additionally comprise an optical communication interface analogous to the optical communication interface 130 of the medical imaging device 100. The medical imaging device 100 may comprise a dedicated receiver analogous to the dedicated receiver of the camera system 200.

In the variant of Fig. 1, the medical imaging device 100 further comprises a first controller 140 coupled to the storage 124 and configured to control the optical communication interface 130. The medical imaging device 100 further comprises a second controller 142 coupled to the storage 124 and the first controller 140. The second controller 142 is configured to control imaging of the medical imaging device 100. The first controller 140 and the storage 124 form a dedicated computation unit (a so-called "smart unit"). The second controller 142 belongs to a core computation unit of the medical imaging device 140 and is configured to control central workflows such as imaging procedures.

The medical imaging device 100 further comprise a power source 150. The power source 150 is configured to power imaging by the medical imaging device 100 and also the optical tracker 120 and the optical communication interface 130.

As explained above, the medical imaging/tracking system 10 further comprises a camera system 200. The camera system 200 comprise a tracking camera 210 (e.g., configured as a stereo camera sensitive in the infrared spectrum). The camera system 200 further comprises a computing device 220 with a processor configured to process image information and other data received by the camera system 200 from the medical imaging device 100 (e.g., including the optical communication signals as described herein).

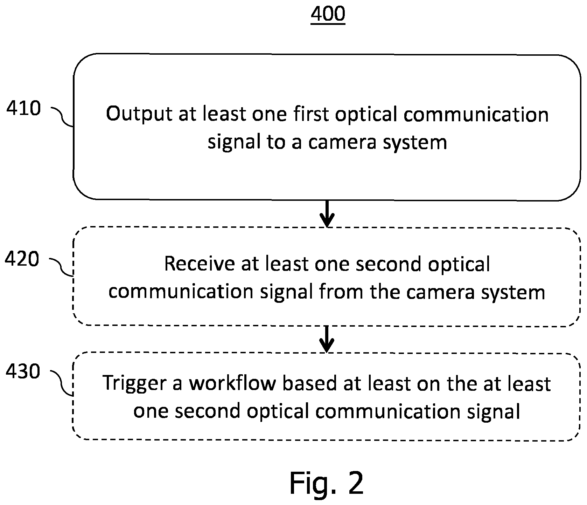

The medical imaging device 100 is configured to communicate with the camera system 200 via communication signals output via the optical communication interface 130 and detected by the tracking camera 210, as will now be described with reference to a flow diagram 400 in Fig. 2. The flow diagram 400 illustrates a method of operating the medical imaging device 100.

The method illustrated in the flow diagram 400 of Fig. 2 comprises a step 410 of outputting, by the imaging device 100, one or more first optical communication signals to the camera system 200. The first optical communication signals are output via the LEDs 132 of the optical communication interface 130.

The first optical communication signals are indicative of one or more items of the calibration information stored in the storage 124 (e.g., the marker locations in the tracker coordinate system COS_tracker and/or one or both of the transformations T_tr_d and T_d_im). Optionally, for example as an alternative or in addition, the one or more first optical communication signals may be indicative of a workflow state of the medical imaging device 100. For example, the medical imaging device 100 may be operable in a scanning workflow that can transit from a scan readiness state to a scan finished state, and vice versa.

The medical imaging device 100 may further be configured to receive one or more second optical communication signals from the camera system 200, as indicated in Fig. 2 by the optional second step 420. To this end, the optical communication interface 130 may be configured with one or more optical receivers. The second optical communication signals may be indicative of a visibility of at least one of the markers 122 of the tracker 120 by the tracking camera 210. As an example, the second optical communication signals may be indicative of a number of markers 122 visible to the tracking camera 120. Additionally or alternatively, the second optical communication signals may be indicative of an instruction to the medical imaging device 100. For example, the second optical communication signals may be indicative of an instruction to trigger a workflow of the medical imaging device 100 (e.g., an imaging workflow or a calibration workflow) or an instruction to transit from one workflow state to another workflow state.

At least one of the controllers 140, 142 of the medical imaging device 100, or a dedicated further controller (not shown), may be configured for processing the received second optical communication signals and to trigger a workflow of the medical imaging device 100 based at least on the second optical communication signals. This is indicated in the optional third step 430 of the flow diagram 400 in Fig. 2. The triggered workflow may comprise a calibration or re-calibration workflow. Alternatively, the triggered workflow may be an imaging workflow for performing a scan and generating patient image data.

Returning to Fig. 1, the tracking camera 210 of the camera system 200 is configured to detect (and track) the markers 122 of the tracker 120. The tracking camera 210 defines a camera coordinate system COS_camera. Based on image information generated by the camera 210, a coordinate transformation T_c_tr between the coordinate system defined by the camera COS_camera and the coordinate system defined by the tracker 130 (i.e., COS_tracker) of the medical imaging device 100 may be determined (e.g., by the computing device 220). For determining the orientation of the coordinate system defined by the tracker 130 (i.e., COS_tracker) in the camera coordinate system COS_camera, the computing device may evaluate the calibration information received in step 410 in terms of the relative marker positions of the tracker 130. This item of calibration information allows the computing device 220 to identify the tracker 130 in the image information acquired by the tracking camera 210 and to determine the location and orientation of tracker 130 in the camera coordinate system COS_camera.

Based on the coordinate transformation T_c_tr together with the item of calibration information indicative of the coordinate transformations T_tr_d and T_d_im, a transformation matrix defining a coordinate transformation T_c_im between the coordinate system defined by the camera 210 COS_camera and the coordinate system defined by the image volume COS_image can be determined. The resulting transformation matrix may be utilized for navigation purposes. As an example, a scan performed by the imaging device 100 (i.e., a resulting three-dimensional patient image) may be registered relative to the patient 300 based on the tracked positions of the patient tracker 310 and the tracker 120 of the medical imaging device 100 and visualized to a surgeon. When the patient is moved relative to the imaging device 100, as detected by a relative movement between the patient tracker 310 and the tracker 120 of the medical imaging device 100, patient image visualization is moved as well. In more detail, as illustrated in Fig. 1, the patient tracker 310 defines a dedicated patient coordinate system COS_patient. A coordinate transformation T_p c between the coordinate system defined by the patient tracker COS_patient and the coordinate system defined by the camera system COS_camera may be determined based on the image information generated by the camera system 200. Based on the coordinate transformations T_p_c and T_c_im, a coordinate transformation T_p_im between the coordinate system defined by the patient tracker 310 and the coordinate system defined by the image volume may be determined and used for visualization and, optionally, navigation purposes.

Additionally, or in the alternative, the position of a surgical instrument (not shown) tracked by the camera system 200 may visualized relative to the visualized patient image. The surgical instrument may then be navigated by the surgeon relative to the patient image. The corresponding navigation routine(s) may be executed by the computing device 220 of the camera system 200, which then constitutes a camera-based navigation system, or a computing device of a dedicated navigation computer.

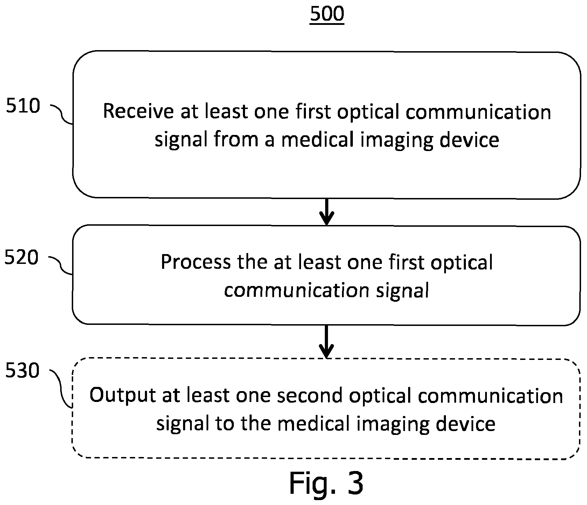

The camera system 200 is configured to communicate with the optical communication interface 130 of the medical imaging device 100 as will now be described with reference to Fig. 3 and a flow diagram 500 illustrating a method for operating the camera system 200. The camera system 200 may comprise a dedicated optical communication interface (not shown) for communicating with the optical communication interface 130 of the medical imaging device 100. In certain variants, the camera system 200 extracts the optical communication signals output by the optical communication interface 130 of the medical imaging device 100 from image information acquired by the tracking camera 210.

The method illustrated in the flow diagram of Fig. 3 comprises a step 510 of receiving one or more first optical communication signals from the medical imaging device 100 as described herein. In a further step 520, the camera system 200 (i.e., the computing device 220) processes the received first optical communication signals. In an optional third step, and at least in part in reaction to the received first optical communication signals, the camera system 200 outputs one or more second optical communication signals to the medical imaging device 100, e.g., for triggering a workflow of the medical imaging device as described herein.

In view of the methods described with reference to Figs. 2 and 3, the embodiments described above provide for an efficient communication of calibration information and other data between the medical imaging device 100 and the camera system 200 for navigation purposes and other procedures, such as triggering of workflows of the medical imaging device 100. In some variants, the calibration information pertaining to the tracker 130 of the medical imaging device 100 can already be determined and stored in the storage 124 at the manufacturing stage. This means that only sporadic or event-driven (re-)calibrations are needed in the operating room. Some workflows may be triggered automatically, e.g., a re-calibration may be automatically triggered in case a calibration date is older than a threshold or sequence of scans may be subsequently triggered based on the workflow state indicated by the optical communication interface 130.

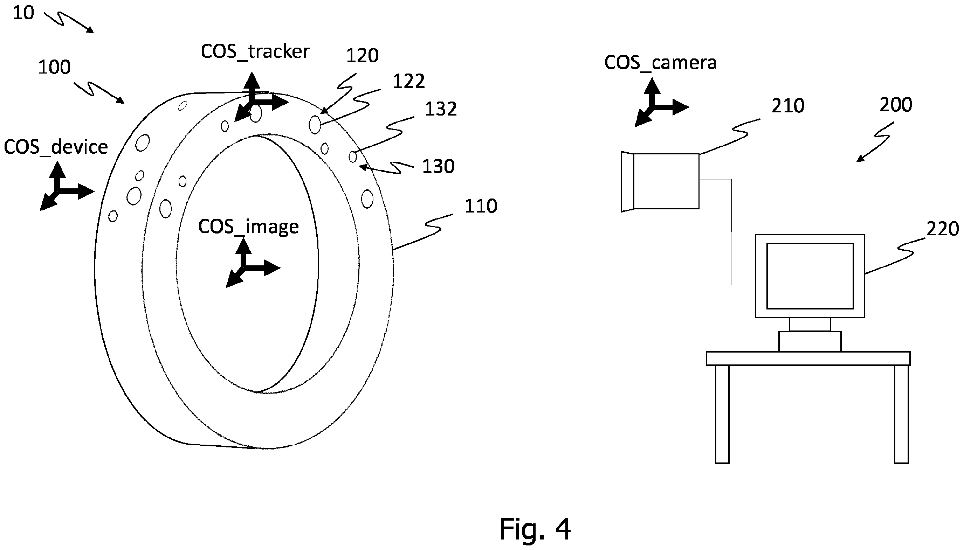

Fig. 4 schematically shows the system 10 of Fig. 1, wherein the medical imaging device 100 is rotated compared to Fig. 1. As can be seen in Fig. 4, some of the optically detectable markers 122 of the tracker 120 and some of the LEDs 132 of the optical communication interface may be located at a top surface of the housing 110 of the medical imaging device 100 to ensure that at least one of the markers 122 and at least one of the LEDs 132 is always visible by the camera 210 of the camera system 200 in all technically feasible orientations between the imaging device 100 and the camera 210.

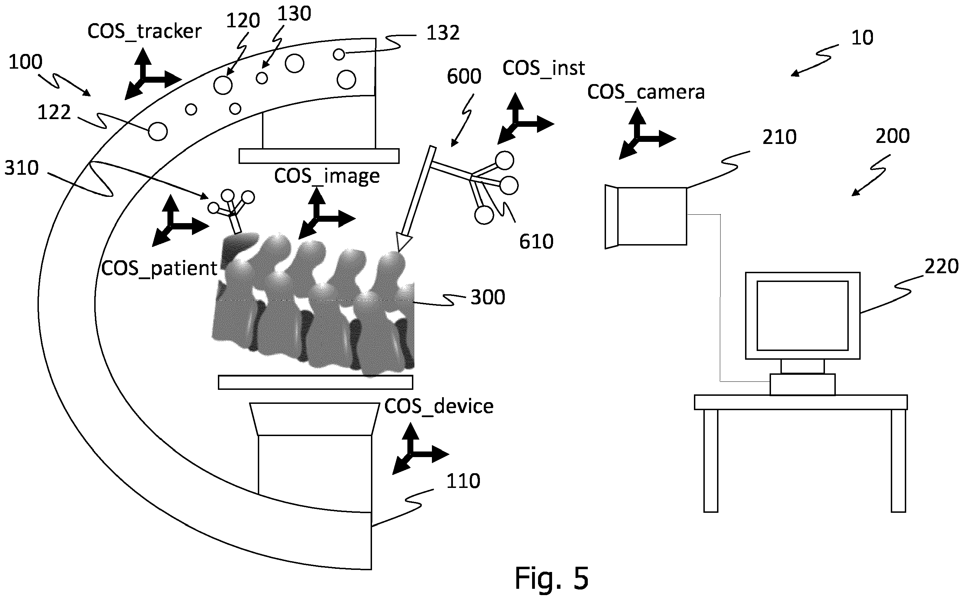

Fig. 5 schematically shows a system 10 comprising a second variant of the medical imaging device 100 (the camera system 200 of Fig. 1 has not changed). The medical imaging device 100 of Fig. 5 comprises a rigid housing 110 that is realized in form of a C-arm.

Further, compared to the system of Fig. 1, a trackable medical instrument 600, e.g., a pointer or a drill, with an instrument tracker 610 (and an associated instrument coordinate system COS_inst) is shown. The instrument tracker 610, the patient tracker 310 and the tracker 120 of the medical imaging device 100 may all be tracked simultaneously by the tracking camera 210 of the camera system 200. Based on the tracking data and the coordinate transformations described with reference to Fig. 1, the camera system 200 may generate navigation instructions for navigating the medical instrument 600 relative a patient image acquired by the medical imaging device 100, as generally described above.

The features described in relation to the exemplary embodiments shown in the drawings can be readily combined to result in different embodiments. It is apparent, therefore, that the present disclosure may be varied in many ways. Such variations are not to be regarded as a departure from the scope of the invention as defined by the claims appended hereto.

## Claims

1. A medical imaging device (100) comprising
a non-stationary housing (110);
an optical tracker (120) configured to be detected by a camera system (200) and comprising a plurality of optically detectable markers (122) attached to the housing (110);
at least one storage (124) configured to store calibration information relating to the optical tracker (120); and
an optical communication interface (130) configured to output to the camera system (200) at least one first optical communication signal indicative of the calibration information.

2. The medical imaging device (100) according to claim 1, wherein
the calibration information is indicative of at least one of relative positions between the optically detectable markers (122) and locations at which the optically detectable markers (122) are attached to the housing (110).

3. The medical imaging device (100) according to claim 1 or 2, wherein
the medical imaging device (100) defines an image volume, and wherein the calibration information is indicative of a positional relationship between the optical tracker (120) and the image volume.

4. The medical imaging device (100) according to claim 3, wherein
each of the optical tracker (120) and the image volume defines a respective coordinate system, and the positional relationship between the optical tracker (120) and the image volume is defined based on a coordinate transformation between the coordinate system defined by the optical tracker (120) and the coordinate system defined by the image volume.

5. The medical imaging device (100) according to claim 4, wherein
the medical imaging device (100) defines a coordinate system, and wherein the coordinate transformation between the coordinate system defined by the optical tracker (120) and the coordinate system defined by the image volume is based on coordinate transformations between
i) the coordinate systems defined by the medical imaging device (100) and the optical tracker (120); and
ii) the coordinate systems defined by the medical imaging device (100) and the image volume.

6. The medical imaging device (100) according to claim 5, wherein
the transformation between the coordinate systems defined by the medical imaging device (100) and the optical tracker (120) is determined based on image data generated with the medical imaging device (100) and indicative of a calibration phantom device.

7. The medical imaging device (100) according to any preceding claim, wherein
the medical imaging device (100) is configured to transit a workflow, and wherein the at least one first optical communication signal is indicative of a workflow state of the medical imaging device (100).

8. The medical imaging device (100) according to any preceding claim, wherein
the optical communication interface (130) is configured to receive at least one second optical communication signal.

9. The medical imaging device (100) according to claim 8, wherein
the at least one second optical communication signal is indicative of at least one of:
i) a visibility of at least one of the plurality of optically detectable markers (122); and
ii) an instruction to the medical imaging device (100).

10. The medical imaging device (100) according to claim 9, wherein
the medical imaging device (100) is configured to trigger a workflow in response to the at least one second optical communication signal, wherein
the triggered workflow comprises at least one of:
i) a calibration or a re-calibration of the medical imaging device (100); and
ii) a generation of image data with the medical imaging device (100).

11. The medical imaging device (100) according to claim 10 when depending on any of claims 3 to 6, wherein
the at least one second optical communication signal is indicative of a position of the optical tracker (120) within a coordinate system defined by the camera system (200), and wherein the workflow comprises determining a coordinate transformation between the coordinate system defined by the image volume and the coordinate system defined by the camera system (200) based at least on the at least one second optical communication signal and the positional relationship between the optical tracker (120) and the image volume.

12. The medical imaging device (100) according to any preceding claim, further comprising:
a first controller (140) coupled to the storage (124) and configured to control the optical communication interface (130); and
a second controller (142) coupled to at least one of the storage (124) and the first controller (140), the second controller (142) being configured to control imaging of the medical imaging device (100).

13. The medical imaging device (100) according to claim 12 when depending on any of claims 7 to 11, wherein
the first controller (140) is configured to at least one of process the at least one second optical communication signal and transmit the received or processed at least one second optical communication signal to the second controller (142).

14. The medical imaging device (100) according to any preceding claim, further comprising:
a power source (150) configured to power the medical imaging device (100) and at least one of the optical tracker (120) and the optical communication interface (130).

15. The medical imaging device (100) according to claim 14, wherein
the power source (150) comprises an energy transmitting element configured to inductively transmit power.

16. The medical imaging device (100) according to any preceding claim, wherein
the medical imaging device (100) is a C arm or computer tomography, CT, scanner.

17. The medical imaging (100) device according to any preceding claim, wherein
the housing (110) comprises a plurality of surfaces, wherein at least two of the surfaces comprise at least one of the plurality of the optically detectable markers (122).

18. A system (10) comprising:
the medical imaging device (100) according to any of claims 1 to 17; and
a camera system (200) configured to detect the optical tracker (120) and to communicate with the optical communication interface (130); and
a processor coupled to the camera system (200) and configured for processing the at least one first optical communication signal indicative of calibration information, wherein processing of the at least one first optical communication signal comprises determining at least one of:
i) a calibration state of the medical imaging device (100);
ii) a coordinate transformation between a coordinate system defined by the camera system (200) and a coordinate system defined by an image volume of the medical imaging device (100); and
iii) at least one second optical communication signal indicative of an instruction to the medical imaging device (100).

19. A method (400) for operating a medical imaging device (100), wherein the medical imaging device (100) comprises a non-stationary housing (110); an optical tracker (120) configured to be detected by a camera system (200) and comprising a plurality of optically detectable markers (122) attached to the housing (110); at least one storage configured to store calibration information relating to the optical tracker (120); and an optical communication interface (130) configured to output to the camera system (200) at least one optical communication signal indicative of the calibration information, the method comprising:
outputting (410), via the optical communication interface, the at least one optical communication signal to the camera system (200).

20. A method (500) for operating a camera system (200), wherein the camera system (200) comprises at least one camera (210) configured to detect an optical tracker (120) of a medical imaging device (100) and an optical communication interface configured to receive, from the medical imaging device (100), at least one first optical communication signal indicative of calibration information relating to an optical tracker (120) attached to a non-stationary housing (110) of the medical imaging device (100), the method comprising:
receiving (510), via the optical communication interface (130), the at least one first optical communication signal from the medical imaging device (100); and
processing (520) the at least one optical communication signal.

21. The method (500) according to claim 20, wherein processing the at least one optical communication signal comprises determining at least one of:
i) a calibration state of the medical imaging device (100);
ii) a coordinate transformation between a coordinate system defined by the camera system (200) and a coordinate system defined by an image volume of the medical imaging device (100); and
iii) at least one second optical communication signal indicative of an instruction to the medical imaging device (100).

22. A computer program product, comprising instructions that, when executed on at least one processor, cause the at least one processor to carry out the method of any of claims 19 to 21.
